# FASCICULE DE BREVET EUROPEEN

(11) **EP 4 436 635 B1**
(45) Date de publication et mention de la délivrance du brevet: **22.04.2026**
(21) Numéro de dépôt: 22809531.1
(22) Date de dépôt: 21.11.2022
(51) Int. Cl.: A61M 5/315

(54) **DISPOSITIF DE SUPPORT DE SERINGUE, DISPOSITIF D'INJECTION ET INSTALLATION ROBOTISÉE D'INTERVENTION CHIRURGICALE**
VORRICHTUNG ZUM HALTEN EINER SPRITZE, INJEKTIONSVORRICHTUNG UND ROBOTEREINHEIT FÜR CHIRURGISCHE EINGRIFFE
SYRINGE SUPPORT DEVICE, INJECTION DEVICE AND ROBOTIC SURGICAL INTERVENTION INSTALLATION

(30) Priorité: 24.11.2021 FR 2112467
(43) Date de publication de la demande: 02.10.2024
(73) Titulaire: Collin, 92220 Bagneux (FR)
(72) Inventeur: MAZALAIGUE, Stéphane, 92220 Bagneux (FR)
(74) Mandataire: Bonnet, Michel
(86) Numéro de dépôt international: PCT/IB2022/061213
(87) Numéro de publication internationale: WO 2023/094963

(56) Documents cités:
- FR-A- 1 101 575
- US-A1- 2012 283 656
- US-A1- 2015 300 391

## Description

La présente invention concerne un dispositif de support de seringue, un dispositif d'injection à support et seringue, ainsi qu'une installation robotisée d'intervention chirurgicale comportant un tel dispositif d'injection.

Il existe de nombreux types de supports de seringues pour différents usages, dont certains visent à apporter davantage de précision et de sécurité dans le geste d'injection d'un produit par seringue.

L'invention s'applique plus particulièrement à un dispositif de support de seringue comportant :
- une première portion de logement d'un piston de seringue, à extrémité proximale de guidage du piston de seringue en translation le long d'un axe principal ; et
- une deuxième portion de support d'un corps de seringue, dans le prolongement de la première portion, à extrémité distale de maintien du corps de seringue de manière à le placer le long de l'axe principal de translation du piston de seringue.

Un tel dispositif de support est par exemple divulgué dans le document de brevet FR 2 616 221 A1. Il permet l'injection automatique de produits médicamenteux mais reste à usage manuel pour l'armement à l'aide d'une tige-guide. Or il existe aujourd'hui des installations robotisées d'intervention chirurgicale à bras articulé qui permettent de se libérer d'une contrainte en termes de disponibilité des mains du chirurgien. Mais le dispositif de support du document précité n'est pas prévu pour cela et l'adaptation nécessaire d'un robot d'intervention chirurgicale pour libérer les mains du chirurgien à l'aide d'un tel dispositif reste difficile à envisager.

Un autre dispositif de support est divulgué dans le document de brevet EP 1 457 220 B1. Un arbre fileté parallèle au piston de seringue et lié par filetage à celui-ci permet son accompagnement en translation le long de l'axe longitudinal du corps de seringue. Cet arbre fileté est compatible avec une motorisation, le rendant implicitement actionnable dans une installation robotisée d'intervention chirurgicale. Mais là encore, cela nécessite une adaptation spécifique de l'installation robotisée.

US 2012/0283656 A1 divulgue un dispositif destiné à faciliter la manipulation d'une seringue. Le dispositif comprend un micromètre qui peut être relié au piston de la seringue par l'intermédiaire d'un châssis de fixation.

FR 1 101 575 divulgue un dispositif automatique pour la manipulation de seringues.

Il peut ainsi être souhaité de prévoir un dispositif de support de seringue qui permette de s'affranchir des problèmes et contraintes précitées, notamment en étant conçu pour un raccordement mécanique simple à un robot d'intervention chirurgicale.

Il est donc proposé un dispositif de support de seringue comportant :
- une première portion de logement d'un piston de seringue, à extrémité proximale de guidage du piston de seringue en translation le long d'un axe principal ; et
- une deuxième portion de support d'un corps de seringue, dans le prolongement de la première portion, à extrémité distale de maintien du corps de seringue de manière à le placer le long de l'axe principal de translation du piston de seringue.
dans lequel l'extrémité proximale de guidage de la première portion du dispositif de support comporte :
- des moyens de fixation à un bras articulé de robot ; et
- un trou traversant de passage d'une tige coulissante robotisée du bras articulé de robot, ce trou traversant étant disposé de manière à inclure un axe de translation pour la tige coulissante confondu avec l'axe principal de translation du piston de seringue lorsque le corps de cette dernière est maintenu en place dans la deuxième portion.

Ainsi, la configuration proposée est aisément raccordable à un bras articulé de robot à l'intérieur duquel est prévu une tige déplaçable en translation par coulissement, ce qui est aujourd'hui notamment le cas du robot décrit dans l'article de Nguyen et al, intitulé « From conception to application of a tele-operated assistance robot for middle ear surgery », publié dans Surgical Innovation,volume 19(3), pages 241-251, septembre 2012. Elle présente en outre l'avantage de combiner les deux fonctions de raccordement (en incluant les moyens de fixation) et de guidage (grâce au trou traversant permettant le passage d'une tige coulissante robotisée pour entraîner le piston de seringue en translation) en une seule et même extrémité du dispositif de support.

De façon optionnelle, le trou traversant est de forme cylindrique centrée sur l'axe principal.

De façon optionnelle également, l'extrémité proximale de guidage comporte un manchon cylindrique dans une face externe duquel sont formés les moyens de fixation et à l'intérieur duquel est formé le trou traversant. Ainsi, les deux fonctions de raccordement et de guidage sont avantageusement remplies de façon compacte par ce manchon cylindrique.

De façon optionnelle également, les moyens de fixation comportent une gorge de verrouillage creusée longitudinalement dans la face externe du manchon cylindrique.

De façon optionnelle également, la deuxième portion comporte en outre, à son extrémité proche de la première portion, un logement de maintien d'une ailette de préhension du corps de seringue. Le positionnement stable et précis de la seringue s'en trouve alors conforté.

De façon optionnelle également, l'extrémité distale de maintien du corps de seringue de la deuxième portion est munie d'un clips ou d'une butée semi-circulaire.

Il est également proposé un dispositif d'injection à support et seringue comportant :
- une seringue à corps de seringue et piston de seringue coulissant le long d'un axe commun du corps et du piston de seringue ; et
- un dispositif de support de seringue selon la présente invention ;
   dans lequel la seringue est disposée dans le dispositif de support de sorte que l'axe commun du corps et du piston de seringue est confondu avec l'axe de translation qu'inclut le trou traversant de passage de tige coulissante robotisée.

Il est également proposé une installation robotisée d'intervention chirurgicale comportant :
- un robot muni d'un bras articulé déplaçable sur commande électronique et pourvu d'une extrémité libre munie d'une tige coulissante robotisée à axe de translation ; et
- un dispositif d'injection selon la présente invention ;
   dans laquelle l'extrémité libre du bras articulé du robot présente des moyens de fixation complémentaires coopérant avec les moyens de fixation de l'extrémité proximale de guidage de la première portion du dispositif de support du dispositif d'injection, de manière à fixer le dispositif d'injection sur l'extrémité libre du bras articulé tout en engageant l'axe de translation de la tige coulissante robotisée dans le trou traversant de l'extrémité proximale de guidage du dispositif de support.

De façon optionnelle, les moyens de fixation complémentaires sont intermédiaires entre l'extrémité libre du bras articulé et le dispositif de support et comportent un dispositif à cylindre creux pourvu de deux bagues de serrage, l'une pour fixation à l'extrémité libre du bras articulé, l'autre pour fixation à l'extrémité proximale de guidage de la première portion du dispositif de support.

De façon optionnelle également, la seringue est en outre pourvue d'un embout à l'extrémité distale libre de son corps et d'une aiguille creuse à pointe formant un point pivot de l'installation robotisée.

L'invention sera mieux comprise à l'aide de la description qui va suivre, donnée uniquement à titre d'exemple et faite en se référant aux dessins annexés dans lesquels :
- la [Fig.1] représente schématiquement en perspective la structure générale d'un dispositif de support de seringue, selon un mode de réalisation de l'invention,
- la [Fig.2] représente schématiquement en vue de dessus la structure générale d'un dispositif d'injection à support et seringue, selon un mode de réalisation de l'invention,
- la [Fig.3] représente schématiquement en vue de dessus la structure générale d'un dispositif d'injection à support et seringue, selon une variante du mode de réalisation de la [Fig.2],
- la [Fig.4] représente schématiquement en coupe la structure générale d'une extrémité libre d'un bras articulé de robot d'intervention chirurgicale, munie d'une tige coulissante robotisée, et
- la [Fig.5] représente schématiquement en perspective la structure générale d'une Installation robotisée d'intervention chirurgicale, selon un mode de réalisation de l'invention.

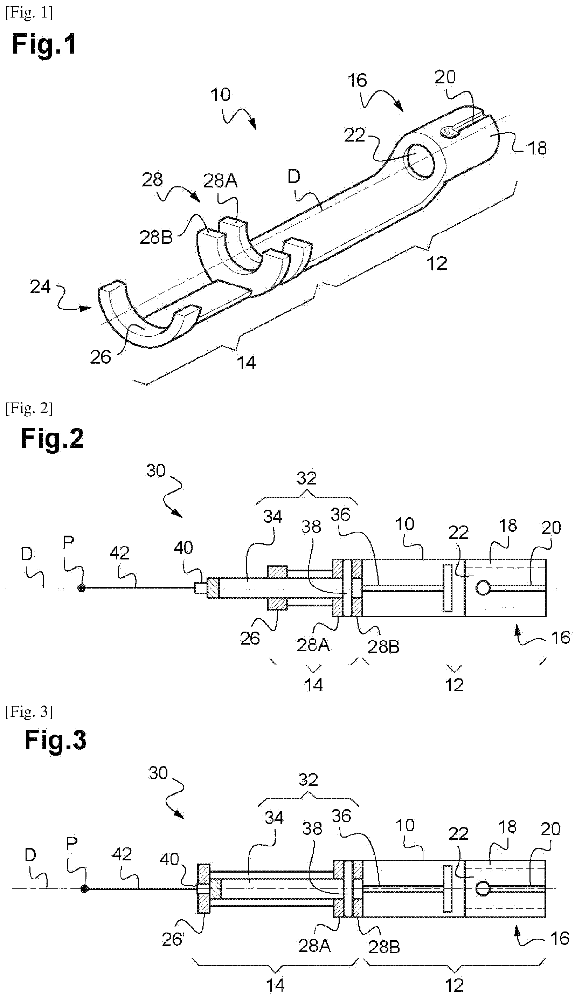

Le dispositif 10 de support de seringue représenté schématiquement en perspective sur la [Fig.1] comporte une première portion 12 de logement d'un piston de seringue et une deuxième portion 14 de support d'un corps de seringue. La deuxième portion 14 est disposée dans le prolongement de la première portion 12.

La première portion 12 est principalement en forme de gouttière mais présente à une extrémité proximale libre 16 un manchon cylindrique 18, surélevé par rapport au fond de la gouttière et venu de matière avec cette dernière. Ce manchon cylindrique 18 présente tout d'abord des moyens 20 de fixation à un bras articulé de robot. Ils sont par exemple matérialisés par une simple gorge de verrouillage 20 de section semi-circulaire creusée longitudinalement dans la face externe du manchon cylindrique 18 et se terminant optionnellement par une cavité partiellement sphérique de plus grande profondeur, en conformité avec l'enseignement du brevet FR 2 998 344 B1.

Le manchon cylindrique 18 présente en outre un trou traversant cylindrique 22 formé par alésage selon une direction longitudinale parallèle au fond de la gouttière. Ce trou traversant 22 est destiné à permettre le passage d'une tige coulissante robotisée du bras articulé de robot auquel le manchon cylindrique 18 est destiné à être fixé, selon un axe principal de translation D qui est l'axe de révolution de sa forme cylindrique. Cet axe principal de translation D est destiné à être celui du piston de seringue lorsque le corps de cette dernière est maintenu en place dans la deuxième portion 14 de sorte que le manchon cylindrique 18 permet le guidage du piston de seringue dans le corps de seringue par poussée de la tige coulissante robotisée le long de l'axe D.

La deuxième portion 14 est également principalement en forme de gouttière, par exemple plus fine que celle de la première portion 12. Elle présente une extrémité distale libre 24 de maintien du corps de seringue de manière à le placer le long de l'axe principal de translation D. Cette extrémité distale libre 24 est ainsi par exemple munie d'un clips semi-circulaire 26, formé sur la gouttière de la deuxième portion 14 et dans lequel vient se loger par serrage une partie médiane ou distale du corps de seringue. Le clips 26 est centré autour de l'axe principal de translation D. En variante, la deuxième portion 14 peut être rallongée et le clips semi-circulaire 26 peut être remplacé par une butée semi-circulaire à logement configuré pour recevoir, en butée du corps de seringue, un embout de moindre diamètre situé à l'extrémité distale libre du corps de seringue. L'autre extrémité de la deuxième portion 14, proche de la première portion 12, présente un logement 28 de maintien d'une ailette de préhension du corps de seringue. Ce logement 28 prend par exemple la forme de deux demi-bagues parallèles 28A, 28B s'étendant chacune dans un plan orthogonal à l'axe principal de translation D à partir des gouttières des deux portions 12 et 14, entre lesquelles vient se loger l'ailette de préhension du corps de seringue. Les deux demi-bagues 28A et 28B sont elles aussi centrées autour de l'axe principal de translation D et conformées pour maintenir le corps de seringue centré autour de ce même axe.

Le dispositif d'injection 30 représenté schématiquement en vue de dessus sur la [Fig.2] comporte le dispositif de support 10 de la [Fig.1] et une seringue 32 à corps 34 et piston 36. Le corps 34 de seringue est maintenu en position ferme dans la deuxième portion 14 autour de l'axe principal de translation D à l'aide du clips semi-circulaire 26 qui enserre sa partie médiane et du logement 28 qui reçoit son ailette de préhension 38 par enserrement entre les deux demi-bagues parallèles 28A et 28B. Le piston 36 partiellement engagé dans le corps 34 de seringue vient alors logiquement se loger dans la première portion 12 en vis-à-vis du manchon cylindrique 18, c'est-à-dire en étant positionné autour de l'axe principal de translation D face au trou traversant cylindrique 22.

La seringue 32 est en outre pourvue d'un embout 40 de moindre diamètre à l'extrémité distale libre de son corps 34 et d'une aiguille creuse 42 à pointe P pour l'administration d'un produit contenu dans le corps 34 de seringue.

Le dispositif d'injection 30 représenté schématiquement en vue de dessus sur la [Fig.3] illustre la variante selon laquelle la deuxième portion 14 est rallongée et le clips semi-circulaire 26 est remplacé par une butée semi-circulaire 26' à logement configuré pour recevoir, en butée du corps 34 de seringue, l'embout 40 de moindre diamètre.

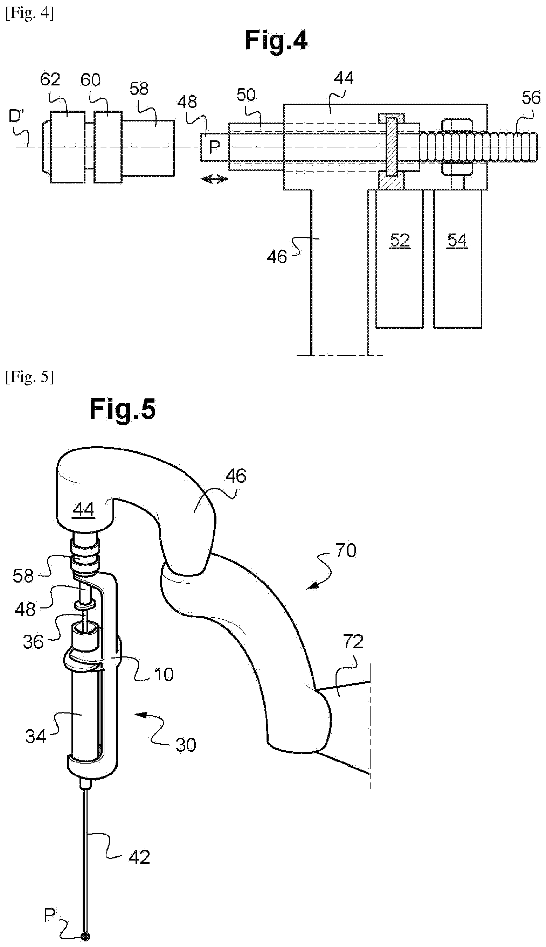

L'extrémité libre 44 d'un bras articulé 46 de robot d'intervention chirurgicale, sur laquelle peut être fixé le dispositif d'injection 30 de la [Fig.2] ou celui de la [Fig.3], est représentée schématiquement en coupe sur la [Fig.4]. Elle est munie d'une tige coulissante robotisée 48 montée libre en translation à l'intérieur d'un manchon 50. L'actionnement en rotation de la tige coulissante robotisée 48 autour d'un axe D' est réalisé à l'aide d'un premier moteur de rotation 52 qui entraîne, de façon connue en soi, le manchon 50 et la tige coulissante robotisée 48. L'actionnement en translation de la tige coulissante robotisée 48 est réalisé à l'aide d'un deuxième moteur de translation 54 qui entraîne, de façon connue en soi également, une tige filetée 56 venant pousser la tige coulissante robotisée 48 le long de l'axe D'.

L'extrémité libre 44 du bras articulé 46 est en outre pourvue de moyens de fixation complémentaires 58 coopérant avec les moyens de fixation 20 de l'extrémité proximale de guidage 16 de la première portion 12 du dispositif de support 10 du dispositif d'injection 30, de manière à fixer ce dernier sur l'extrémité libre 44 du bras articulé 46 tout en engageant l'axe D' de translation de la tige coulissante robotisée 48 dans le trou traversant 22 de l'extrémité proximale de guidage 16 du dispositif de support 10.

Ces moyens de fixation complémentaires 58, intermédiaires entre l'extrémité libre 44 du bras articulé 46 et le dispositif de support 10, comportent par exemple un dispositif à cylindre creux pourvu de deux bagues de serrage 60 et 62, l'une 60 pour fixation à l'extrémité libre 44, l'autre 62 pour fixation au manchon cylindrique 18. Ils sont creux pour laisser passer en translation la tige coulissante robotisée 48. La gorge de verrouillage 20 formée dans le manchon cylindrique 18 permet d'accompagner et guider angulairement l'insertion du dispositif de support 10 dans les moyens de fixation complémentaires 58, comme enseigné dans le brevet FR 2 998 344 B1. Plus généralement, tous moyens de fixation connus et compatibles avec la configuration de bras articulé 46 illustrée sur la [Fig.4] sont envisageables, notamment tous moyens permettant de fixer le dispositif d'injection 30 de telle sorte que son axe principal de translation D soit confondu avec l'axe d'intervention D' du bras articulé 46.

Plus globalement, l'installation robotisée 70 d'intervention chirurgicale représentée schématiquement en perspective sur la [Fig.5] comporte un robot 72 comprenant lui-même au moins un bras articulé dont le bras articulé 46 de la [Fig.4], déplaçable sur commande électronique et pourvu de l'extrémité libre 44 munie de la tige coulissante robotisée 48. Le dispositif d'injection 30 y est représenté monté sur l'extrémité libre 44 à l'aide des moyens de fixation complémentaires 58 de sorte que les axes D et D' se confondent.

Ainsi, la tige coulissante robotisée 48 peut venir en butée contre la tête du piston 36 de la seringue 32 dont le corps 34 est maintenu en position fixe dans le dispositif de support 10. Le corps 34 de seringue, l'aiguille 42 et sa pointe P sont donc fixes par rapport au référentiel de l'extrémité libre 44 du bras articulé 46. Il en résulte que la pointe P de l'aiguille 42 peut former un point pivot de l'installation robotisée 70, c'est-à-dire un point qui peut rester fixe dans le référentiel du robot 72 lorsque celui-ci déplace le bras articulé 46 selon trois axes de rotations différents. En particulier, en chirurgie otologique, une telle configuration du dispositif d'injection 30 est très avantageuse pour une intervention au cours de laquelle le geste chirurgical nécessite une grande précision.

Il apparaît clairement qu'un dispositif de support de seringue, un dispositif d'injection et une installation robotisée d'intervention chirurgicale tels que ceux décrits précédemment permettent le raccordement simple et précis d'une seringue à un bras articulé de robot à l'intérieur duquel est prévu une tige déplaçable en translation par coulissement, pour ensuite pouvoir l'actionner en injection de produit à l'aide de cette tige.

On notera par ailleurs que l'invention n'est pas limitée aux modes de réalisation décrits précédemment. Il apparaîtra en effet à l'homme de l'art que diverses modifications peuvent être apportées aux modes de réalisation décrits ci-dessus, à la lumière de l'enseignement qui vient de lui être divulgué. Notamment, les formes des différents éléments décrits précédemment restent assez libres à partir du moment où les fonctions techniques auxquelles ils sont destinés sont respectées.

## Revendications

1. Dispositif (10) de support de seringue (32) comportant :
- une première portion (12) de logement d'un piston (36) de seringue (32), cette première portion (12) comportant une extrémité proximale (16) de guidage du piston (36) de seringue (34) en translation le long d'un axe principal (D) ; et
- une deuxième portion (14) de support d'un corps (34) de seringue (32), dans le prolongement de la première portion (12), cette deuxième portion (14) comportant une extrémité distale (24) de maintien du corps (34) de seringue (32) de manière à le placer le long de l'axe principal (D) de translation du piston (36) de seringue (32) ;
**caractérisé en ce que** l'extrémité proximale de guidage (16) de la première portion (12) du dispositif de support (10) comporte un manchon cylindrique (18) :
- dans une face externe duquel sont formés des moyens de fixation (20) à un bras articulé de robot par insertion du manchon cylindrique (18) dans des moyens de fixation complémentaires du bras articulé de robot ; et
- à l'intérieur duquel est formé un trou traversant (22) de passage d'une tige coulissante robotisée du bras articulé de robot, ce trou traversant (22) étant disposé de manière à inclure un axe de translation pour la tige coulissante confondu avec l'axe principal (D) de translation du piston (36) de seringue (32) lorsque le corps (34) de cette dernière est maintenu en place dans la deuxième portion (14).

2. Dispositif (10) de support de seringue (32) selon la revendication 1, dans lequel le trou traversant (22) est de forme cylindrique centrée sur l'axe principal (D).

3. Dispositif (10) de support de seringue (32) selon la revendication 1 ou 2, dans lequel :
- la première portion (12) est principalement en forme de gouttière, le manchon cylindrique (18) étant surélevé par rapport au fond de la gouttière et venu de matière avec cette dernière ; et
- le trou traversant (22) est formé par alésage selon une direction longitudinale parallèle au fond de la gouttière.

4. Dispositif (10) de support de seringue (32) selon la revendication 3, dans lequel les moyens de fixation (20) comportent une gorge de verrouillage creusée longitudinalement dans la face externe du manchon cylindrique (18).

5. Dispositif (10) de support de seringue (32) selon l'une quelconque des revendications 1 à 4, dans lequel la deuxième portion (14) comporte en outre, à son extrémité proche de la première portion, un logement (28) de maintien d'une ailette de préhension (38) du corps (34) de seringue (32).

6. Dispositif (10) de support de seringue (32) selon l'une quelconque des revendications 1 à 5, dans lequel l'extrémité distale (24) de maintien du corps (34) de seringue (32) de la deuxième portion (14) est munie :
- d'un clips (26) dans lequel vient se loger par serrage une partie médiane ou distale du corps (34) de seringue (32) ; ou
- d'une butée semi-circulaire (26') à logement configuré pour recevoir, en butée du corps (34) de seringue (32), un embout (40) de moindre diamètre situé à l'extrémité distale libre du corps (34) de seringue (32).

7. Dispositif d'injection (30) à support et seringue comportant :
- une seringue (32) à corps (34) de seringue et piston (36) de seringue coulissant le long d'un axe commun du corps (34) et du piston (36) de seringue (32) ; et
- un dispositif (10) de support de seringue (32) selon l'une quelconque des revendications 1 à 6 ;
dans lequel la seringue (32) est disposée dans le dispositif de support (10) de sorte que l'axe commun du corps (34) et du piston (36) de seringue (32) est confondu avec l'axe de translation qu'inclut le trou traversant (22) de passage de tige coulissante robotisée.

8. Installation robotisée (70) d'intervention chirurgicale comportant :
- un robot (72) muni d'un bras articulé (46) déplaçable sur commande électronique et pourvu d'une extrémité libre (44) munie d'une tige coulissante robotisée (48) à axe de translation (D') ; et
- un dispositif d'injection (30) selon la revendication 7 ;
dans laquelle l'extrémité libre (44) du bras articulé (46) du robot (72) présente des moyens de fixation complémentaires (58) coopérant avec les moyens de fixation (20) de l'extrémité proximale de guidage (16) de la première portion (12) du dispositif de support (10) du dispositif d'injection (30), de manière à fixer le dispositif d'injection (30) sur l'extrémité libre (44) du bras articulé (46) tout en engageant l'axe de translation (D') de la tige coulissante robotisée (48) dans le trou traversant (22) de l'extrémité proximale de guidage (16) du dispositif de support (10).

9. Installation robotisée (70) d'intervention chirurgicale selon la revendication 8, dans laquelle les moyens de fixation complémentaires (58) sont intermédiaires entre l'extrémité libre (44) du bras articulé (46) et le dispositif de support (10) et comportent un dispositif à cylindre creux pourvu de deux bagues de serrage (60, 62), l'une (60) pour fixation à l'extrémité libre (44) du bras articulé (46), l'autre (62) pour fixation à l'extrémité proximale de guidage (16) de la première portion (12) du dispositif de support (10).

10. Installation robotisée (70) d'intervention chirurgicale selon la revendication 8 ou 9, dans laquelle la seringue (32) est en outre pourvue d'un embout (40) à l'extrémité distale libre de son corps (34) et d'une aiguille creuse (42) à pointe (P) formant un point pivot de l'installation robotisée (70).

## Patentansprüche

1. Trägervorrichtung (10) einer Spritze (32), die Folgendes aufweist:
- einen ersten Abschnitt (12) zum Aufnehmen eines Kolbens (36) der Spritze (32), wobei dieser erste Abschnitt (12) ein proximales Führungsende (16) des Kolbens (36) der Spritze (34) zum Verschieben entlang einer Hauptachse (D) aufweist; und
- einen zweiten Abschnitt (14) zum Tragen eines Körpers (34) der Spritze (32) in Verlängerung des ersten Abschnitts (12), wobei dieser zweite Abschnitt (14) ein distales Ende (24) zum Halten des Körpers (34) der Spritze (32) aufweist, um diesen entlang der Hauptverschiebungsachse (D) des Kolbens (36) der Spritze (32) zu positionieren;
**dadurch gekennzeichnet, dass** das proximale Führungsende (16) des ersten Abschnitts (12) der Trägervorrichtung (10) eine zylindrische Hülse (18) aufweist:
- an deren Außenseite Befestigungsmittel (20) an einem Robotergelenkarm durch Einsetzen der zylindrischen Hülse (18) in ergänzende Befestigungsmittel des Robotergelenkarms gebildet sind; und
- innerhalb dessen ein Durchgangsloch (22) zum Durchführen eines Roboterschiebestabs des Robotergelenkarms gebildet ist, wobei dieses Durchgangsloch (22) so angeordnet ist, dass es eine Translationsachse für den Schiebestab einschließt, die mit der Hauptachse (D) zum Verschieben des Kolbens (36) der Spritze (32) übereinstimmt, wenn der Körper (34) dieser in dem zweiten Abschnitt (14) gehalten wird.

2. Trägervorrichtung (10) einer Spritze (32) nach Anspruch 1, wobei das Durchgangsloch (22) zylindrisch geformt und auf der Hauptachse (D) zentriert ist.

3. Trägervorrichtung (10) einer Spritze (32) nach Anspruch 1 oder 2, wobei:
- der erste Abschnitt (12) überwiegend die Form einer Rinne hat, wobei die zylindrische Hülse (18) gegenüber dem Boden der Rinne erhöht ist und mit diesem aus einem Stück besteht; und
- das Durchgangsloch (22) durch Bohrung in einer Längsrichtung parallel zu dem Boden der Rinne gebildet wird.

4. Trägervorrichtung (10) einer Spritze (32) nach Anspruch 3, wobei die Befestigungsmittel (20) eine längs in die Außenseite der zylindrischen Hülse (18) ausgehöhlte Verriegelungsnut aufweisen.

5. Trägervorrichtung (10) einer Spritze (32) nach einem der Ansprüche 1 bis 4, wobei der zweite Abschnitt (14) ferner an seinem Ende nahe des ersten Abschnitts eine Aufnahme (28) zum Halten eines Griffflügels (38) des Körpers (34) der Spritze (32) aufweist.

6. Trägervorrichtung (10) einer Spritze (32) nach einem der Ansprüche 1 bis 5, wobei das distale Ende (24) zum Halten des Körpers (34) der Spritze (32) des zweiten Abschnitts (14) mit Folgendem ausgestattet ist:
- einem Clip (26), in dem sich ein mittlerer oder distaler Teil des Körpers (34) der Spritze (32) durch Festklemmen befindet; oder
- einem halbrunden Anschlag (26') mit einer Aufnahme, die so eingerichtet ist, dass sie an dem Anschlag des Körpers (34) der Spritze (32) einen Aufsatz (40) mit einem kleineren Durchmesser eingliedert, der sich an dem freien distalen Ende des Körpers (34) der Spritze (32) befindet.

7. Injektionsvorrichtung (30) mit Halter und Spritze, die Folgendes aufweist:
- eine Spritze (32) mit Spritzenkörper (34) und Spritzenkolben (36), die entlang einer gemeinsamen Achse des Körpers (34) und des Kolbens (36) der Spritze (32) verschiebbar ist; und
- eine Trägervorrichtung (10) der Spritze (32) nach einem der Ansprüche 1 bis 6;
wobei die Spritze (32) in der Trägervorrichtung (10) so angeordnet ist, dass die gemeinsame Achse des Körpers (34) und des Kolbens (36) der Spritze (32) mit der Translationsachse übereinstimmt, die das Durchgangsloch (22) zum Durchführen eines Roboterschiebestabs einschließt.

8. Robotergestützte chirurgische Eingriffsanordnung (70), die Folgendes aufweist:
- einen Roboter (72) mit einem Gelenkarm (46), der elektronisch beweglich ist und mit einem freien Ende (44) versehen ist, das mit einem Roboterschiebestab (48) mit Translationsachse (D') ausgestattet ist; und
- eine Injektionsvorrichtung (30) nach Anspruch 7;
wobei das freie Ende (44) des Gelenkarms (46) des Roboters (72) zusätzliche Befestigungsmittel (58) besitzt, die mit den Befestigungsmitteln (20) des proximalen Führungsendes (16) des ersten Abschnitts (12) der Trägervorrichtung (10) der Injektionsvorrichtung (30) zusammenwirken, so dass die Injektionsvorrichtung (30) an dem freien Ende (44) des Gelenkarms (46) befestigt wird, während die Translationsachse (D') des Roboterschiebestabs (48) in das Durchgangsloch (22) des proximalen Führungsendes (16) der Trägervorrichtung (10) eingreift.

9. Robotergestützte chirurgische Eingriffsanordnung (70) nach Anspruch 8, wobei die ergänzenden Befestigungsmittel (58) zwischen dem freien Ende (44) des Gelenkarms (46) und der Trägervorrichtung (10) angeordnet sind und eine Hohlzylindervorrichtung aufweisen, die mit zwei Klemmringen (60, 62) versehen ist, wobei einer (60) zum Befestigen an dem freien Ende (44) des Gelenkarms (46), der andere (62) zum Befestigen an dem proximalen Führungsende (16) des ersten Abschnitts (12) der Aufnahmevorrichtung (10) dient.

10. Robotergestützte chirurgische Eingriffsanordnung (70) nach Anspruch 8 oder 9, wobei die Spritze (32) ferner mit einem Aufsatz (40) an dem freien distalen Ende ihres Körpers (34) und einer Hohlnadel (42) mit Spitze (P) versehen ist, die einen Drehpunkt der robotergestützten Anordnung (70) bildet.

## Claims

1. A syringe (32) support device (10) comprising:
- a first portion (12) for housing a syringe (32) piston (36), this first portion (12) comprising a proximal end (16) for guiding the syringe (34) piston (36) in translation along a main axis (D); and
- a second portion (14) for supporting a syringe (32) body (34), as an extension in line of the first portion (12), this second portion (14) comprising a distal end (24) for holding the syringe (32) body (34) so as to position it along the main axis (D) of translation of the syringe (32) piston (36);
**characterised in that** the guiding proximal end (16) of the first portion (12) of the support device (10) comprises a cylindrical sleeve (18):
- in an outer surface of which are formed means for fastening (20) to a robot articulated arm by insertion of the cylindrical sleeve (18) into complementary fastening means on the robot articulated arm; and
- inside which is formed a through hole (22) for the passage of a robotic sliding rod of the robot articulated arm, this through hole (22) being arranged so as to include a translation axis for the sliding rod coinciding with the main axis (D) of translation of the syringe (32) piston (36) when the body (34) of the syringe is held in place in the second portion (14).

2. The syringe (32) support device (10) according to claim 1, wherein the through hole (22) is cylindrical in shape and centred on the main axis (D).

3. The syringe (32) support device (10) according to claim 1 or 2, wherein:
- the first portion (12) is mainly gutter-shaped, the cylindrical sleeve (18) being raised above the bottom of the gutter and formed from the same material as the latter; and
- the through hole (22) is formed by boring in a longitudinal direction parallel to the bottom of the gutter.

4. The syringe (32) support device (10) according to claim 3, wherein the fastening means (20) comprise a locking groove carved longitudinally into the outer face of the cylindrical sleeve (18).

5. The syringe (32) support device (10) according to any of the claims from 1 to 4, wherein the second portion (14) further comprises, at its end close to the first portion, a housing (28) for holding a gripping fin (38) of the body (34) of the syringe (32).

6. The syringe (32) support device (10) according to any of the claims from 1 to 5, wherein the distal end (24) for holding the syringe (32) body (34) of the second portion (14) is equipped with:
- a clip (26) wherein a middle or distal part of the syringe (32) body (34) is clamped in place; or
- a semi-circular stop (26') with a housing configured to receive, at the stop of the syringe (32) body (34), a tip (40) of smaller diameter located at the free distal end of the syringe (32) body (34).

7. An injection device (30) with support and syringe comprising:
- a syringe (32) with a syringe body (34) and a syringe piston (36) sliding along a common axis of the body (34) and the piston (36) of the syringe (32); and
- the syringe (32) support device (10) according to any of the claims from 1 to 6;
wherein the syringe (32) is arranged in the support device (10) such that the common axis of the body (34) and piston (36) of the syringe (32) coincides with the axis of translation included in the through hole (22) for the passage of the robotic sliding rod.

8. A robotic surgical intervention installation (70) comprising:
- a robot (72) equipped with an articulated arm (46) that can be moved by electronic control and provided with a free end (44) equipped with a robotic sliding rod (48) with a translation axis (D'); and
- the injection device (30) according to claim 7;
wherein the free end (44) of the articulated arm (46) of the robot (72) has complementary fastening means (58) cooperating with the fastening means (20) of the guiding proximal end (16) of the first portion (12) of the support device (10) of the injection device (30), so as to fasten the injection device (30) to the free end (44) of the articulated arm (46) while engaging the translation axis (D') of the robotic sliding rod (48) in the through hole (22) of the guiding proximal end (16) of the support device (10).

9. The robotic surgical intervention installation (70) according to claim 8, wherein the complementary fastening means (58) are located between the free end (44) of the articulated arm (46) and the support device (10) and comprise a hollow cylinder device provided with two clamping rings (60, 62), one (60) for fastening to the free end (44) of the articulated arm (46), the other (62) for fastening to the guiding proximal end (16) of the first portion (12) of the support device (10).

10. The robotic surgical intervention installation (70) according to claim 8 or 9, wherein the syringe (32) is further provided with a tip (40) at the free distal end of its body (34) and a hollow needle (42) with a point (P) forming a pivot point of the robotic installation (70).
